# EUROPEAN PATENT SPECIFICATION

(11) **EP 1 904 225 B1**
(45) Date of publication and mention of the grant of the patent: **29.02.2012**
(21) Application number: 06747655.6
(22) Date of filing: 26.05.2006
(51) Int. Cl.: B01J 19/18, B01F 7/18, B01J 19/20

(54) **CYLINDRICAL REACTOR FOR THE TREATMENT OF AN AGITATED MATERIAL COMPOSITION FOR A PREDETERMINED RETENTION TIME**
ZYLINDRISCHER REAKTOR ZUR BEHANDLUNG EINER GERÜHRTEN STOFFZUSAMMENSETZUNG ÜBER EINE VORGEGEBENE RETENTIONSZEIT
RÉACTEUR CYLINDRIQUE POUR LE TRAITEMENT D' UNE COMPOSITION MATÉRIELLE AGITÉE PENDANT UNE PÉRIODE DE RÉTENTION PRÉDÉTERMINÉE

(30) Priority: 27.05.2005 NO 20052540
(43) Date of publication of application: 02.04.2008
(73) Proprietor: Aglen, Lars, 7168 Lysöysund (NO)
(72) Inventor: ULGENES, Yngve, N-7160 Bjugn (NO)
(74) Representative: Jennings, Michael John
(86) International application number: PCT/NO2006/000196
(87) International publication number: WO 2006/126891

(56) References cited:
- EP-A2- 1 245 599
- US-A- 3 539 732
- US-A- 4 138 544

## Description

The present invention concerns a reactor for the treatment of a material composition comprising at least two components, such as organic material that is subjected to treatment with an enzyme or a mixture of enzymes.

### Background

When e.g. subjecting organic material to enzymatic treatment in the form of hydrolysis (decomposition) it is a provision for a successful result that the temperature of the material and the time during which the material is contacted (contact period) by the enzymes are controlled. Too long or too short contact periods will both be negative for the product resulting from the process and may lead to problems for the further treatment of the material and/ or may be negative with respect to the quality of the resulting product from a manufacturing process. Correct contact period is a central issue in this respect.

When utilizing industrial enzymes for hydrolysis or other forms of enzymatic processes, the relevant enzymes are added to a raw material. When the enzymes have been added, it is important that the mixture is continuously agitated to ensure good contact between the enzymes and the raw material. At the same time it is important that the material is contacted by the enzymes for a defined period of time. When this period of time has been reached it is equally important that the enzymatic decomposition is interrupted to avoid the excessive decomposition. This is normally obtained by heating the mixture of raw material and enzymes to a degree by which the enzymes are destroyed (deactivated).

A corresponding challenge occurs for a number of other chemical processes where a homogenous mixture of the entering components and a highly controlled reaction period is vital, a reaction period that neither can be significantly longer or significantly shorter than the optimal period if desired quality of the end product shall be obtainable.

The simplest way to ensure correct contact period is to use batch type reactors. In batch reactors a fixed volume (a tank or the like) is held at certain conditions for a certain period of time and thereafter the process is interrupted. In enzymatic processes heating is as mentioned commonly used to deactivate enzymes. In an industrial scale production large volumes are typically processed and if run as a batch process it will be difficult to heat the entire batch sufficiently rapid. An alternative of running a high number of small batches would lead to comparatively high equipment costs.

There are also other disadvantages related to batch processing compared to continuous processes irrespective of whether enzymes are involved or not. One such disadvantage is the high number of start up and shut-down of the process which is labour intensive and harder to automatize than continuous processes. In addition the conditions may typically vary unfavourably during start up and shut-down.

The ideal solution is a continuous flow of a homogenously mixed raw material wherein the process is deactivated after a certain period of time. Allowing a continuous flow of raw material to pass through a large "complete mix" tank is not a good solution since the contact period for the individual components will be very hard to control. In a continuous production process it is desirable to obtain conditions that are as similar to a plug (piston) flow as possible. This means that any partial volume entering a process line should be discharged from the other end of the process line as the same partial volume. From well described hydraulic principles this can largely be obtained by arranging a number of complete mix chambers in a series. If a high number of such complete mix reactors are arranged in a series, the resulting flow of material therethrough would be quit close to a plug flow. It can be shown mathematically that by arranging a high number of complete mix volumes (tanks) in a series, an almost ideal plug flow is obtained. This means that with such a design a partial volume that enters the process at the inlet will leave the process (be discharged) as the same partial volume having passed through the apparatus under good agitation and with a defined retention time (contact period).

US 4,138,544 A discloses an improvement in the method of preparing a linear polycondensate of high molecular weight wherein a precondensate is initially formed and thereafter subjected to a one-step melt condensation in a vacuum while cleavage products are removed therefrom, the reaction mass being exposed in a thin layer by means of a stirrer to the reaction conditions, the improvement residing in employing as the precondensate a molten precondensate having an average condensation degree of at least 3, said precondensate being introduced into the melt condensation zone continuously while the cleavage products are driven off at the particular optimum, product-specific condensation temperature, the contents of the melt condensation zone being subjected to the action of a rotary stirrer until the melt has a melt viscosity of 1000 to 15000 poises and continuously withdrawing molten polycondensate in accordance with the speed of the reaction. Also disclosed is an apparatus in which the polycondensation is effected including an offset withdrawal outlet containing an auger which can enter a funnel disposed between the outlet and the polycondensation zone. Also disclosed is an upright multi-chamber vessel in which the precondensate is formed wherein the chambers are disposed one over the other. Some of the upper chambers are equipped with means for maintaining a normal or elevated pressure while some of the lower chambers are equipped with means for imposing a partial vacuum on the contents.

### Objectives

It is an object of the present invention to provide a reactor which allows advantages related to batch type processes.

It is furthermore an object to be able to do so with means that are convenient and inexpensive in industrial scale.

It is hereunder a deviated objectto provide a reactor treating the entering material as a plug flow.

### The invention

The objects mentioned above are fulfilled by a reactor as defined in claim 1.

Preferred embodiments of the invention are disclosed by the dependent claims.

The material to be treated in the reactor is sometimes referred to as "raw material", sometimes as "the material". Below two reactor according to the present invention shall be describes more in detail shown by the enclosed drawings.

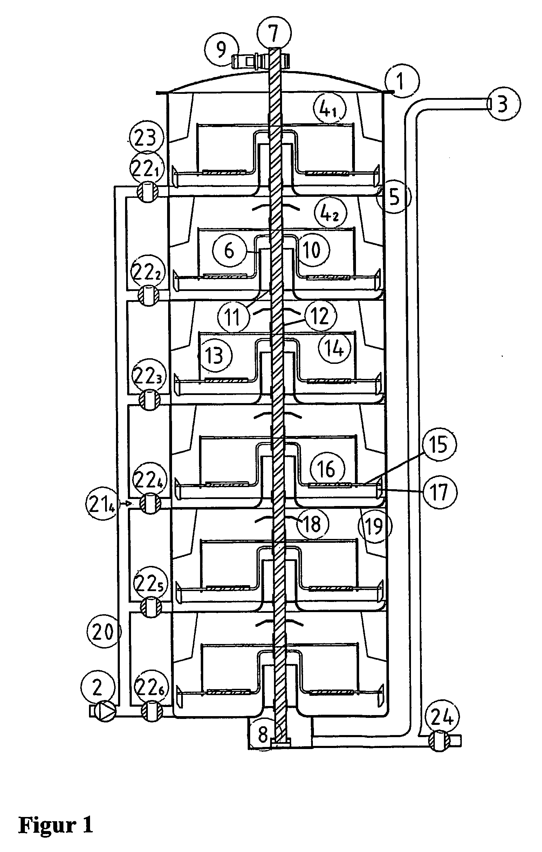

Fig. 1 is a cross sectional side view of a reactor comprising five factor chambers, not falling within the scope of the claims.
Fig. 2 is a partially sectioned perspective drawing of an arbitrary reactor chamber of the reactor in
Fig. 3 illustrates schematically the material movement within a reactor chamber, not according to the invention.
Fig. 4 is a side sectional view of a reactor chamber, according to the invention.

The reactor comprises a vertical, cylindrical tank 1 with an inlet 2 for raw material and on outlet 3 for discharging material after completed reaction. The vertical tank 1 is divided into several chambers 4ᵢ by circular plates 5ᵢ that are tightly fit against the inner wall of the tank 1 and thus separate the chambers 4ᵢ from one another. The index "i" indicates that an arbitrary element among several principally equal elements is discussed. Such indices are not consistently used in the drawings, however. The number of chambers 4ᵢ that the tank is divided into is decided by the number of circular plates 5ᵢ. At the centre of each circular plate 5ᵢ there is a conduit 6ᵢ that extends from the plate and to a height within the chamber, e.g. to half the height of the chamber. This conduit connects one chamber to the next so that material being treated may pass through the conduit to an adjacent chamber 4ᵢ₊₁ beneath. The cross-section of the conduit 6ᵢ must be adapted to the type of raw material being treated. The conduit should, however, not be wider than allowing raw material to pass through by means of a slight overpressure, thereby avoiding movement back and forth between the chambers. In general the direction of movement of material in the reactor is downwards from above.

A centric shaft 7 being extends from top to bottom of the shown reactor 1 held by a bearing 8 at the reactor bottom. The shaft is rotated by means of a motor 9 with gear at the top pf the tank. The shaft 7 is preferably assembled from several sections by means of connecting couplings 12 with one coupling per chamber. The shaft 7 passes through a guiding muff 11 arranged in the conduits 6 between each chamber.

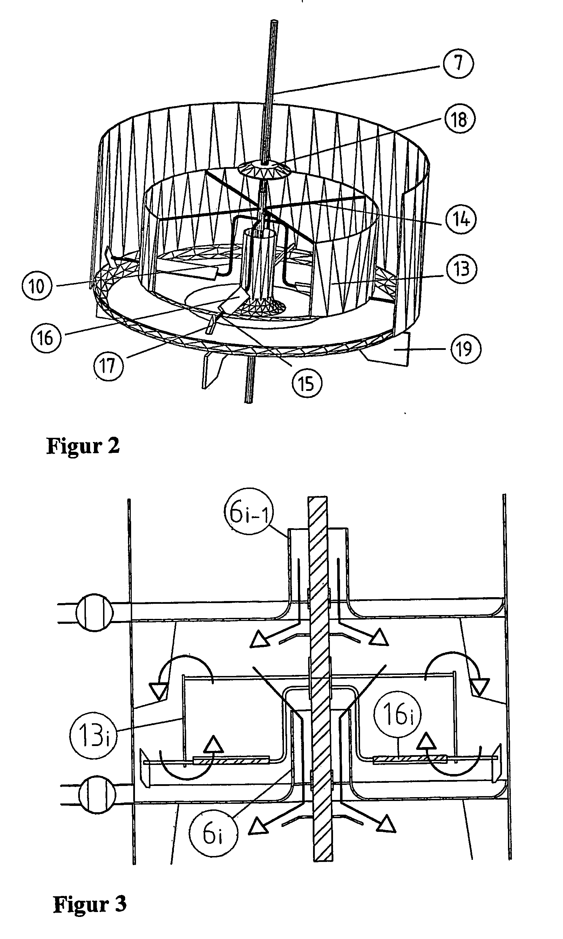

A paddle mechanism 10ᵢ is connected to the shaft 7 in each chamber to facilitate movement and agitation of the raw material/ material in the chambers. A preferred embodiment of the paddle mechanism is shown in Fig. 2 where one chamber is shown partially opened and in perspective. The shown paddle mechanism 10ᵢ comprises a ring-shaped member 13ᵢ which is attached to the shaft 7 by two sets of bars 14 and 15(like spokes in a wheel). To the lower bar 15 an agitating member 16i is attached having the shape of a tilted (propeller) blade. The lower bars 15 extend radially beyond the ring-shaped member 13ᵢ and end in outer, aslant blades 17ᵢ that are closely adjacent to the tank wall.

To the shaft 7 is also attached a deflector 18ᵢ which is positioned straight below the conduit 6ᵢ₋₁ of the above adjacent chamber 4ᵢ₋₁.

To the lower side of each circular plate 5ᵢ₋₁ that separates the chambers 4ᵢ₋₁ and 4ᵢ there are normally attached a set of vertical plates 19ᵢ that serve to decelerate circular flow of material in the chamber 4ᵢ. In the upper chamber 4ᵢ the vertical plates 19₁ are attached to the reactor top in the absence of a circular plate.

On the outside of the reactor 1 and connected to the inlet 2 a vertical tubing 20 is arranged having branches 21ᵢ provided with valves 22ᵢ that may be opened and closed separately, connected to each chamber 4ᵢ.

The reactor 1 size is adapted so that a defined flow rate corresponds to a desired contact period or reaction period for the raw material passing through the reactor. By dividing the reactor int a number of chambers the flow of material approaches a plug flow. With increasing number of chambers the flow comes closer to an ideal plug flow.

Below the mode of operation of the reactor as illustrated by the drawings 1-3, is described more closely in connection with a process for enzymatic treatment of a raw material, typically a marine raw material.

When the reactor is in continuous operation (steady state) raw material that immediately before has been mixed with enzymes are being pumped in a continuous flow through inlet 2. In the first chamber the material is thoroughly mixed by the paddle mechanism 10ᵢ and a substantially complete mix is obtained in this chamber. From the first chamber 4₁ the material is passed to the next chamber 4₂ through the conduit 6₁ at the centre of the circular plate 5₁. In this next chamber 4₂ there is also a substantially complete mix ensured by the paddle mechanism 10₂ and so forth. A corresponding amount (volume) treated material is discharged by conduit 3 as feed into the reactor by conduit 2. The raw material to be treated may typically have the form of a dispersion. Since the conduit 6ᵢ opening is at a certain elevation above the bottom of each reactor chamber 4ᵢ, particles that tend to settle out of the dispersion are not able to "short-circuit" the reactor by passing through the reactor more rapid than other material. The diameter of the conduit 6ᵢ is normally chosen small enough to avoid that its extension will decide the level of material in the reactor chambers. The reactor chambers 4ᵢ may thus be full even if the conduits 6ᵢ only extend to half the height of the chambers.

The movement of the raw material in each chamber is shown in Figure 3. The paddle mechanism 10ᵢ in each chamber 4ᵢ rotates with an angular velocity controlled by the motor 9. The agitating member 16ᵢ attached to the lower bar 15ᵢ of the ring-shaped member 13ᵢ will during rotation force the raw material upwards in the chamber. In practice the agitating member 16ᵢ will induce a movement comprising a horizontal as well as a vertical component of movement. Since the ring-shaped member 13ᵢ constitutes a physical barrier between the outer part of the chamber (close to the chamber wall) and the inner part of the chamber (close to its centre) a flow of material as shown in Figure 3 will be established in the chamber. The vertical plates 19ᵢ will retard the horizontal circular component of movement of the raw material. The aslant blades 17ᵢ attached to the lower bar 15ᵢ and moving along the outermost part of the chamber prevent formation of "dead" zones near the reactor's outer wall where particles and other material may settle.

It is important that the rotational speed is high enough to hold particles in the raw material in dispersion. Since the movement of the raw material is generally upwards close to the centre of each chamber (within the ring-shaped member 13_{¡}) a random selection of particles will enter the conduit to the next chamber, leading to a desired statistical retention time. With a significant number of chambers in series as with the illustrated reactor, a flow close to a plug flow is in practice achieved.

The plate 18ᵢ attached to the shaft 7 ensures that the raw material entering a chamber from the chamber 4ᵢ₋₁ above is directed outwards and follows the general flow within the chamber, cf. Figure 3. In this manner material entering a chamber is prevented from passing directly through to the next conduit 6, thereby "short circuiting" the chamber.

When the reactor is started all the chambers are empty. During start-up, that means until all chambers are filled - the external tube 20 is used. In the start-up period all valves 22₁ - 22₅ are open while the valves 23 and 24 are closed. In this way raw material containing enzymes is filled first to the lowermost chamber. During filling of the chambers no material is discharged through outlet 3. During start-up there is in theory neither any exchange of material between chambers.

When all the chambers have been filled, the raw material in the lowermost chamber has had the required treatment period and the valve 23 is opened while the valves 22₁ - 22₅ are all closed. The raw material that thereafter enters the reactor through inlet 2 is directed to the uppermost chamber while material that has received the desired treatment is discharged through outlet 3.

When the reactor is shut-down, material is pumped out through outlet 3 with such a rate that the correct retention time is achieved for the last entered raw material. For the emptying of the reactor the valves 22₁ - 22₅ are opened according to a system ensuring that the chambers are emptied in the right sequence and that the material mainly passes through the respective chambers to obtain the desired retention time.

First valves 22₁ and 22₂ are opened so that there is passage between first and second chamber. Chamber 4ᵢ can not be emptied completely through conduit 6ᵢ since it extends up from the bottom of the chamber. When the level of raw material in chamber 4₁ has reached the top of the conduit the remaining material must be drained through the external tube where valves 22₁ and 22₂ are open. When the level in chamber 4₂ has been reduced valve 22₃ must be opened in order to drain this chamber completely etc. This procedure may be conducted manually or automatically and can be controlled by level sensors in each chamber.

It is preferred that the reactor may be easily dismantled for maintenance and service. The shaft 7 may be withdrawn and dismantled by removing the connecting cpuplings12ᵢ. The paddle mechanisms 10ᵢ arranged in each chamber can be removed and the bottom plates 5ᵢ dividing the reactor in separate chambers 4ᵢ may be lifted out. Maintenance may follow a planned scheme or may be performed if any kind of damage occurs. The outer wall will normally be in one piece only but may also be sectionized, e.g. in sections corresponding to the height of one, two or three chambers 4ᵢ. More normally the reactor outer wall is a one-piece structure among which a number of variants may be chosen to build with modules reactors with a desired number of chambers.

A typical use of the reactor as described above is for the enzymatic treatment of krill or corresponding organisms with a composition allowing the material/ suspension to be a homogenous one for the entire treatment process.

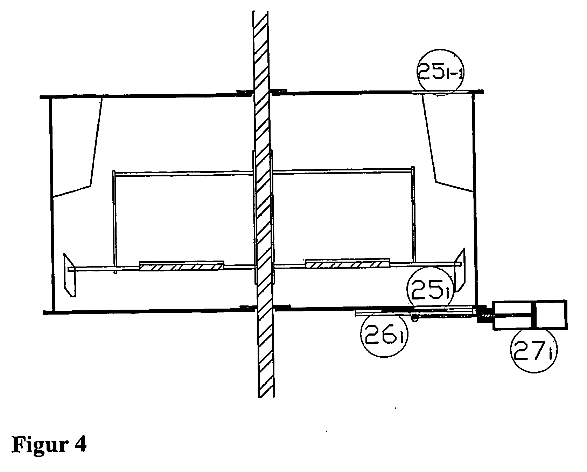

If, however, the raw material is such that the material will separate into a fluid and a solid part during the reaction time the continuous reactor will not work according to the presumptions. This may typically be the case if larger organisms such as fish or crabs are hydrolysed. One relevant area of use is treatment of grounded back and head of fish. During the process in the reactor the protein will be fluid like water. The bones will be released and will due to their higher density sink. In such a case a reactor as shown in Figure 4 is used.

As shown by Figure 4 close to the periphery of each chamber 4ᵢ, or at least in a distance from the periphery, there is arranged an opening 25ᵢ which may be opened or closed by means of a slide damper 26ᵢ which in turn is controlled by means of a hydraulic or pneumatic cylinder 27ᵢ which in turn is controlled by a motor (not shown). According to the invention there is no central opening between the individual chambers 4.

Material is continuously fed to the first chamber 4₁. The mass is agitated while the chamber is filled up. After a certain period of time the slide damper 26₁ is opened and the mass drained batchwise to the next chamber 4₂.

The agitation continuous and the batch proceeds further down the reactor to allow room for the next batch entering from above. In this manner complete mix volumes are allowed to be arranged in series. The time difference between first and last entrance will be maintained during the retention period.

Compared to the continuous reactor a higher number of chambers are required to obtain the same effect (close approximation to a plug flow).

## Claims

1. Cylindrical reactor (1) for continuous treatment of an agitated material composition comprising at least two components, comprising a number of reactor chambers (4ₙ) arranged in a mainly vertical column separated by bottom plates (5ₙ) while transportation of the material composition from an arbitrary reactor chamber (4₁) during stationary conditions is arranged to take place to the adjacent chamber below (4ᵢ₊₁), each reactor chamber (4ᵢ) being provided with a paddle mechanism (10ᵢ), **characterized in that** the paddle mechanism (10₁) comprises a ring-shaped member (13ᵢ) which is concentric with the reactor chamber and has a vertical extension, and at least one movable agitating member (16ᵢ) which is arranged to induce a vertical component of movement to the material in the chamber, wherein the transportation from a chamber (4ᵢ') to the next chamber (4ᵢ₊₁') is arranged to take place periodically through an opening (25ᵢ) with a slide damper (26ᵢ) in the bottom plate (5ᵢ') of each chamber.

2. Reactor (1) as claimed in claim 1, **characterized in that** the ring-shaped member (13ᵢ) has a vertical extension corresponding to approximately half the height of the chamber and is arranged so that the vertical distance from a reactor bottom plate (5ᵢ) to the lower edge of the ring-shaped member (13ᵢ) is about the same as the vertical distance between the reactor chamber's top and the upper edge of the ring-shaped member (13ᵢ).

3. Reactor (1) as claimed in claim 1 or claim 2, **characterized in that** the movable agitating member (16ᵢ) the shape of an aslant plate arranged to rotate within the ring-shaped member (13ᵢ) in the reactor chamber (4₁).

4. Reactor (1) as claimed in any one of the preceding claims, **characterized in that** the paddle mechanism (10₁) in each reactor chamber (4ᵢ) is arranged to be powered by means of a mainly vertical shaft (7) that extends through and is concentrically with the reactor chambers.

5. Reactor (1) as claimed in claim 4, **characterised in that** the ring-shaped member (13ᵢ) is attached to the shaft (7) by means of upper bars (14ᵢ) and lower bars (15ᵢ) and that the agitating member (16ᵢ) is attached to a lower bar (15ᵢ) for during rotation to induce an upward component of movement of the material composition within the ring-shaped member (13₁) in the reactor chamber (4₁) and a downward component of movement of the material composition between the ring-shaped member and the reactor wall.

6. Reactor (1) as claimed in any one of the preceding claims, **characterized in that** the paddle mechanism (10ᵢ) also comprises at least one aslant blade (17ᵢ) arranged close to the wall of the reactor chamber (4ᵢ) to prevent formation of dead zones along the wall.

7. Reactor (1) as claimed in any one of the preceding claims, **characterized in that** each of the reactor chambers (4ᵢ) is provided with stationary, vertical plates (19ᵢ) that extend downwards from the bottom plate (5ᵢ₋₁) of the chamber (4ᵢ₋₁) above to reduce circular movement of the material composition in the reactor chamber (4ᵢ).

8. Reactor (1) as claimed in any one of the preceding claims, **characterized in that** the transition from bottom to reactor wall in each chamber is rounded.

9. Reactor (1) as claimed in any one of the preceding claims, **characterized in that** the internal reactor components are comprised by modules with removable bottom plates (5ᵢ), removable paddle mechanisms (10ᵢ) and a removable and optionally dividable shaft (7).

10. Reactor (1) as claimed in any one of the preceding claims, **characterized in that** the reactor externally comprises or is attached to a supply system for raw material which in addition to a top valve (23) and a bottom valve (24) comprises valves (22ᵢ) level with the bottom of each reactor chamber (4ᵢ) to allow consistent treatment of the material also during start-up and shut-down.

## Patentansprüche

1. Zylindrischer Reaktor (1) für kontinuierliche Behandlung einer gerührten Stoffzusammensetzung, die mindestens zwei Komponenten umfasst, umfassend eine Zahl von Reaktorkammern (4ₙ), die in einer hauptsächlich vertikalen Säule angeordnet sind, getrennt durch Bodenplatten (5ₙ), während Transport der Stoffzusammensetzung von einer beliebigen Reaktorkammer (4ᵢ) im stationären Zustand angeordnet ist, um zu der angrenzenden Kammer unterhalb (4ᵢ₊₁) zu erfolgen, wobei jede Reaktorkammer (4ᵢ) mit einem Flügelmechanismus (10ᵢ) versehen ist, **dadurch gekennzeichnet, dass** der Flügelmechanismus (10ᵢ) ein ringförmiges Glied (13ᵢ) umfasst, das mit der Reaktorkammer konzentrisch ist und eine vertikale Verlängerung aufweist, und mindestens ein bewegbares Rührglied (16ᵢ), das angeordnet ist, um eine vertikale Bewegungskomponente in dem Stoff in der Kammer herbeizuführen, wobei der Transport von einer Kammer (4_{i'}) zu der nächsten Kammer (4_{i+1'}) angeordnet ist, um periodisch durch eine Öffnung (25ᵢ) mit einer Schieberklappe (26ᵢ) in der Bodenplatte (5ᵢ') jeder Kammer stattzufinden.

2. Reaktor (1) nach Anspruch 1, **dadurch gekennzeichnet, dass** das ringförmige Glied (13ᵢ) eine vertikale Verlängerung aufweist, die mit ungefähr der halben Höhe der Kammer korrespondiert, und so angeordnet ist, dass die vertikale Distanz von einer Reaktor-Bodenplatte (5ᵢ) zu der unteren Kante des ringförmigen Glieds (13ᵢ) etwa dasselbe beträgt wie die vertikale Distanz zwischen der Oberseite der Reaktorkammer und der oberen Kante des ringförmigen Glieds (13ᵢ).

3. Reaktor (1) nach Anspruch 1 oder Anspruch 2, **dadurch gekennzeichnet, dass** das bewegbare Rührglied (16ᵢ) die Form einer schrägen Platte aufweist [A1], die angeordnet ist, um sich innerhalb des ringförmigen Glieds (13ᵢ) in der Reaktorkammer (4ᵢ) zu drehen.

4. Reaktor (1) nach einem der vorstehenden Ansprüche, **dadurch gekennzeichnet, dass** der Flügelmechanismus (10ᵢ) in jeder Reaktorkammer (4ᵢ) angeordnet ist, um mittels einer hauptsächlich vertikalen Welle (7), die sich durch die Reaktorkammern erstreckt und damit konzentrisch ist, angetrieben zu werden.

5. Reaktor (1) nach Anspruch 4, **dadurch gekennzeichnet, dass** das ringförmige Glied (13ᵢ) mittels oberer Stäbe (14ᵢ) und unterer Stäbe (15ᵢ) an der Welle (7) angebracht ist und dass das Rührglied (16ᵢ) an einem unteren Stab (15ᵢ) angebracht ist, um während der Drehung eine Aufwärts-Bewegungskomponente der Stoffzusammensetzung innerhalb des ringförmigen Glieds (13ᵢ) in der Reaktorkammer (4ᵢ) und eine Abwärts-Bewegungskomponente der Stoffzusammensetzung zwischen dem ringförmigen Glied und der Reaktorwand herbeizuführen.

6. Reaktor (1) nach einem der vorstehenden Ansprüche, **dadurch gekennzeichnet, dass** der Flügelmechanismus (10ᵢ) außerdem mindestens ein schräges Blatt (17ᵢ) umfasst, das nahe der Wand der Reaktorkammer (4ᵢ) angeordnet ist, um die Bildung von toten Zonen entlang der Wand zu verhindern.

7. Reaktor (1) nach einem der vorstehenden Ansprüche, **dadurch gekennzeichnet, dass** jede der Reaktorkammern (4ᵢ) mit stationären vertikalen Platten (19ᵢ) versehen ist, die sich von der Bodenplatte (5ᵢ₋₁) der Kammer (4ᵢ₋₁) oberhalb nach unten erstrecken, um kreisförmige Bewegung der Stoffzusammensetzung in der Reaktorkammer (4ᵢ) zu reduzieren.

8. Reaktor (1) nach einem der vorstehenden Ansprüche, **dadurch gekennzeichnet, dass** der Übergang von Boden zu Reaktorwand in jeder Kammer gerundet ist.

9. Reaktor (1) nach einem der vorstehenden Ansprüche, **dadurch gekennzeichnet, dass** die internen Reaktorkomponenten sich aus Modulen mit entfernbaren Bodenplatten (5ᵢ), entfernbaren Flügelmechanismen (10ᵢ) und einer entfernbaren und optional teilbaren Welle (7) zusammensetzen.

10. Reaktor (1) nach einem der vorstehenden Ansprüche, **dadurch gekennzeichnet, dass** der Reaktor extern ein Zuführungssystem für Rohstoffe umfasst oder daran angeschlossen ist, das zusätzlich zu einem oberen Ventil (23) und einem unteren Ventil (24) Ventile (22ᵢ) höhengleich mit dem Boden jeder Reaktorkammer (4ᵢ) umfasst, um gleichbleibende Behandlung des Stoffs auch während Inbetriebnahme und Außerbetriebnahme zu gestatten.

## Revendications

1. Réacteur cylindrique (1) pour le traitement continu d'une composition agitée comprenant au moins deux composants, comprenant un certain nombre de chambres de réaction (4ₙ) disposées dans une colonne principalement verticale séparée par des plaques de fond (5ₙ) tandis que le transport de la composition d'une chambre de réaction arbitraire (4₁) pendant des conditions stables est agencé de façon à avoir lieu vers la chambre adjacente en dessous (4ᵢ₊₁), chaque chambre de réaction (4ᵢ) étant pourvue d'un mécanisme à pale (10ᵢ), **caractérisé en ce que** ce mécanisme à pale (10ᵢ) comprend un élément en forme d'anneau (13ᵢ) qui est concentrique avec la chambre de réaction et qui a un prolongement vertical, et au moins un élément d'agitation mobile (16ᵢ) qui est agencé de façon à induire une composante verticale de mouvement à la matière dans la chambre, dans lequel le transport d'une chambre (4ᵢ') à la chambre suivante (4ᵢ₊₁') est agencé de façon à avoir lieu périodiquement à travers une ouverture (25ᵢ) avec un registre à coulisse (26ᵢ) dans la plaque de fond (5₁') de chaque chambre.

2. Réacteur (1) selon la revendication 1, **caractérisé en ce que** l'élément en forme d'anneau (13ᵢ) a un prolongement vertical correspondant à environ la moitié de la hauteur de la chambre et est disposé de manière à ce que la distance verticale entre une plaque de fond du réacteur (5ᵢ) et le bord inférieur de l'élément en forme d'anneau (13ᵢ) soit à peu près la même que la distance verticale entre le sommet de la chambre de réaction et le bord supérieur de l'élément en forme d'anneau (13ᵢ).

3. Réacteur (1) selon la revendication 1 ou la revendication 2, **caractérisé en ce que** l'élément d'agitation mobile (16ᵢ) [A1] a la forme d'une plaque disposée en biais et agencée de façon à tourner à l'intérieur de l'élément en forme d'anneau (13ᵢ) dans la chambre de réaction (4ᵢ)

4. Réacteur (1) selon l'une quelconque des revendications précédentes, **caractérisé en ce que** le mécanisme à pale (10₁) dans chaque chambre de réaction (4ᵢ) est agencé de façon à être mû au moyen d'un arbre principalement vertical (7) qui s'étend à travers les chambres de réaction et qui est concentrique avec celles-ci.

5. Réacteur (1) selon la revendication 4, **caractérisé en ce que** l'élément en forme d'anneau (13ᵢ) est attaché à l'arbre (7) au moyen de barres supérieures (14ᵢ) et de barres inférieures (15ᵢ) et **en ce que** l'élément d'agitation (16ᵢ) est attaché à une barre inférieure (15ᵢ) de façon à induire, pendant la rotation, une composante de mouvement de la composition dirigée vers le haut à l'intérieur de l'élément en forme d'anneau (13ᵢ) dans la chambre de réaction (4ᵢ) et une composante de mouvement de la composition dirigée vers le bas entre l'élément en forme d'anneau et la paroi du réacteur.

6. Réacteur (1) selon l'une quelconque des revendications précédentes, **caractérisé en ce que** le mécanisme à pale (10ᵢ) comprend aussi au moins une lame disposée en biais (17ᵢ) à proximité de la paroi de la chambre de réaction (4ᵢ) pour empêcher la formation de zones mortes le long de la paroi.

7. Réacteur (1) selon l'une quelconque des revendications précédentes, **caractérisé en ce que** chacune des chambres de réaction (4ᵢ) est pourvue de plaques verticales immobiles (19ᵢ) qui s'étendent vers le bas depuis la plaque de fond [5₁₋₁) de la chambre (4ᵢ₋₁) au-dessus pour réduire le mouvement circulaire de la composition dans la chambre de réaction (4ᵢ).

8. Réacteur (1) selon l'une quelconque des revendications précédentes, **caractérisé en ce que** la transition depuis le fond jusqu'à la paroi du réacteur dans chaque chambre est arrondie

9. Réacteur (1) selon l'une quelconque des revendications précédentes, **caractérisé en ce que** les éléments internes du réacteur sont constitués par des modules avec des plaques de fond (5ᵢ) amovibles, des mécanismes à pale (10ᵢ) amovibles et un arbre (7) amovible et facultativement divisible.

10. Réacteur (1) selon l'une quelconque des revendications précédentes, **caractérisé en ce que** ce réacteur comprend extérieurement, ou est attaché à, un système d'alimentation en matière brute qui, en plus d'une soupape supérieure (23) et d'une soupape inférieure (24) comporte des soupapes (22ᵢ) de niveau avec le fond de chaque chambre de réaction (4ᵢ) pour permettre le traitement uniforme de la matière aussi pendant le démarrage et l'arrêt.
